(19) Europäisches Patentamt
European Patent Office
Office européen des brevets

(11) **EP 1 482 739 A1**

(12) **EUROPÄISCHE PATENTANMELDUNG**

(43) Veröffentlichungstag:
01.12.2004  Patentblatt 2004/49

(51) Int Cl.7: **H04N 7/18**

(21) Anmeldenummer: **04004910.8**

(22) Anmeldetag: **03.03.2004**

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL LT LV MK**

(30) Priorität: **30.05.2003  DE 20308463 U**

(71) Anmelder:
• **Armbruster, Siegfried**
  **79312 Emmendingen (DE)**
• **Emminger, Ralf**
  **81543 München (DE)**

(72) Erfinder:
• **Armbruster, Siegfried**
  **79312 Emmendingen (DE)**
• **Emminger, Ralf**
  **81543 München (DE)**

(74) Vertreter: **Weber, Dieter, Dr. et al**
  **Weber, Seiffert, Lieke**
  **Taunusstrasse 5a**
  **65183 Wiesbaden (DE)**

(54) **Elektronische Sehhilfe**

(57)    Die Erfindung betrifft eine elektronische Sehhilfe für Sehbehinderte.

Um eine verbesserte elektronische Sehhilfe bereitzustellen, schlägt die Erfindung vor, daß eine von einer Halteeinrichtung (8) gehaltene Kamera (7) und ein Projektor (4) vorgesehen sind, der das von der Kamera (7) erfaßte Bild in Echtzeit vergrößert auf einer Projektionsfläche (6) abbildet. Dabei ist es besonders vorteilhaft, wenn die elektronische Sehhilfe eine Bedieneinheit (11) zur Fernbedienung von Einstellungen an der Kamera (7) und dem Projektor (4) aufweist. Besonders günstig ist es weiterhin, wenn bei der elektronischen Sehhilfe die Kamera (7) eine digitale Kamera ist und der Projektor (4) einen digitalen Videoeingang aufweist.

FIGUR 2

**Beschreibung**

Die Erfindung betrifft eine elektronische Sehhilfe für Sehbehinderte, insbesondere zur besser erkennbaren Darstellung von Text und Bildern aller Art.

Sehbehinderte Menschen benötigen zur Erkennung von Text- und Bildmaterial oft eine vergrößerte Darstellung. Bekannt sind rein optische Hilfsmittel, die maximal durch ein- oder mehrlinsige Anordnungen eine ca. 12-fache Vergrößerung ermöglichen. Ein Vorteil der rein optischen Hilfsmittel liegt in der mobilen Einsatzmöglichkeit. Ein wesentlicher Nachteil liegt in der Einschränkung des nutzbaren Sehfeldes, das umgekehrt proportional zur Vergrößerung ist.

Eine weitere Möglichkeit zur vergrößerten Darstellung für Sehbehinderte bieten elektronische Systeme, bei denen das Objekt durch eine Kamera aufgenommen wird und nach einer elektronischen Bildbearbeitung auf einem TV- oder Computermonitor abgebildet wird.

Der Vorteil gegenüber der rein optischen Vergrößerung besteht darin, daß das Bild zusätzlich zur optischen Vergrößerung verändert werden kann. So kann zum Beispiel der Kontrast verstärkt, eine Kontrastumkehr vorgenommen oder die Hintergrundfarbe geändert werden. Durch diese zur Vergrößerung zusätzlichen optischen Effekte kann das Erkennen von Schriftbildern oder Gegenständen für den Sehbehinderten weiter erleichtert werden.

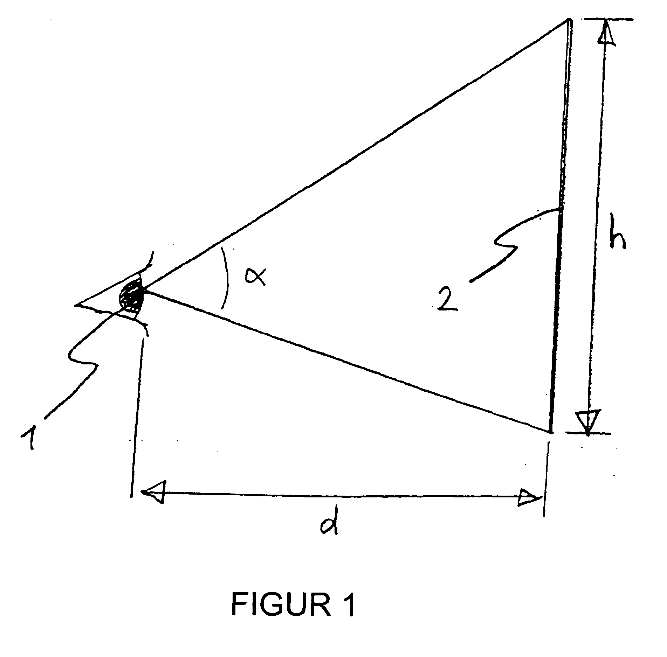

Der Nachteil bei der oben beschriebenen bekannten elektronischen Sehhilfe (Bildschirmlesegerät) besteht darin, daß die nutzbare Bildfläche und damit auch der Betrachtungswinkel durch die Abmessungen des abbildenden Monitors stark eingeschränkt ist. Figur 1 veranschaulicht die Definition des Betrachtungswinkels α, der durch den Abstand d des Betrachters 1 von der Bildfläche 2 und die Abmessungen h der Bildfläche 2 durch die folgende Beziehung näherungsweise definiert ist:

$$\alpha = 2 \arctan (h/2d)$$

Aufgabe der vorliegenden Erfindung ist es, eine weiter verbesserte elektronische Sehhilfe bereitzustellen.

Diese Aufgabe wird gelöst durch eine elektronische Sehhilfe nach Anspruch 1. Weiterbildungen der Erfindung sind in den Unteransprüchen angegeben.

Ein Vorteil ist, daß der Betrachtungswinkel für die erfindungsgemäße Sehhilfe größer als der der bekannten elektronischen oder rein optischen Sehhilfen ist.

Die erfindungsgemäße elektronische Sehhilfe unterscheidet sich von den bislang auf dem Markt befindlichen im wesentlichen dadurch, daß das Bild des zu vergrößernden Objekts 3 nicht mit Hilfe eines Monitors (TV-Bildschirm oder PC-Monitor), sondern durch einen Projektor 4 entweder direkt auf eine Wand 5 oder auf eine Projektionsleinwand abgebildet wird. Durch diese Besonderheit kann die Fläche, die der vergrößerten Abbildung zur Verfügung steht, in Abhängigkeit vom Abstand zwischen Projektor 4 und Projektionsfläche 6 gegenüber den bekannten elektronischen Sehhilfen erheblich vergrößert werden. Gleichzeitig mit der Fläche der Abbildung vergrößert sich auch der Betrachtungswinkel α. Die Wahrnehmung von Text, Bildern oder Gegenständen wird für den Sehbehinderten dadurch erheblich erleichtert.

Zweckmäßig ist es dabei, wenn die Kamera eine Digitalkamera mit einer ausreichend großen Anzahl von Bildpunkten ist. Dies ermöglicht es, Detailausschnitte aus den abzubildenden Objekten heraus zu vergrößern, ohne an Bildqualität zu verlieren.

Bevorzugt wird dabei eine Ausführungsform der Erfindung, bei welcher der Projektor ein Beamer mit einem digitalen Signaleingang ist. Dabei kann der verwendete Beamer entsprechend den Anforderungen des Nutzers in Hinblick auf die Helligkeit, den Geräuschpegel, die Bildqualität oder auch die Kosten ein CRT- bzw. Kathodenstrahl-Beamer, ein LCD- bzw. Flüssigkristall-Beamer oder auch ein DLP- bzw. Spiegel-Beamer sein. Dabei ist die Liste der genannten zur Verfügung stehenden Beamer nicht abschließend, und insbesondere sollen auch zukünftige vorteilhafte Entwicklungen mit dem Erfindungsgedanken kombiniert werden können.

Die elektronsiche Sehhilfe weist zudem den Vorteil auf, daß sie zugleich als Projektor vo n Fernseh-, Video- und Computerbildern genutzt werden kann.

Weitere Merkmale und Zweckmäßigkeiten der Erfindung ergeben sich aus der Beschreibung eines bevorzugten Ausführungsbeispiels anhand der Figuren. Von den Figuren zeigen:

Figur 1    eine Veranschaulichung des Betrachtungswinkels.

Figur 2    eine schematische Darstellung der erfindungsgemäßen Sehhilfe.

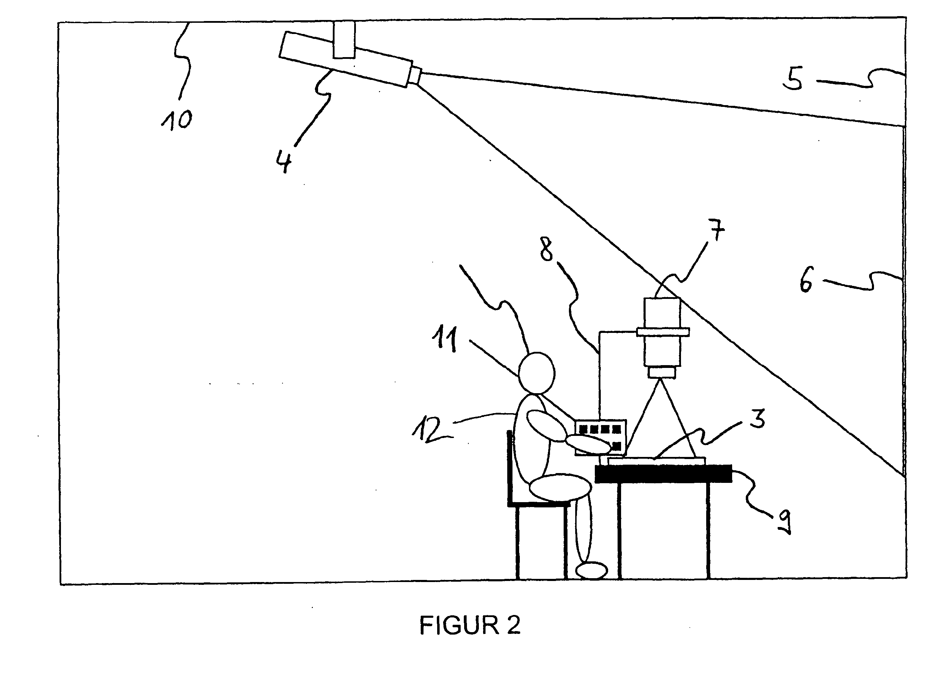

Figur 2 zeigt eine schematische Darstellung eines Ausführungsbeispiels der erfindungsgemäßen elektronischen Sehhilfe. Die elektronische Sehhilfe besteht aus einer Kamera 7, die mit einem Stativ 8 über einer Tischplatte 9 gehalten wird, einem an der Decke befestigten Projektor 4 und einer Bedieneinheit 11.

Die Kamera 7 ist eine digitale Videokamera mit Autofokus und wird durch ein Stativ 8 oberhalb eines auf dem Tisch befindlichen, zu betrachtenden Objekts gehalten. Zur besseren Handhabung ist die Kamera 7 derart mit dem Stativ 8 verbunden, daß sie in alle Richtungen gerichtet werden kann und zudem höhenverstellbar ist. Dadurch können auch weiter entfernte Gegenstände abgebildet werden. Das Stativ 8 ist bei dieser Ausführungsform als Stehstativ ausgebildet, das einfach auf den Tisch gestellt wird.

Der Projektor (Beamer) 4 bildet das von der digitalen Kamera 7 erfaßte Bild des zu betrachtenden Objekts 3 in Echtzeit auf die Projektionsfläche 6 durch Projektion ab. Über eine entsprechende Montagevorrichtung ist er platzsparend an der Decke 10 montiert und auf die dem Sehbehinderten gegenüberliegende Projektionsfläche 6 gerichtet. Dabei wird als Projektionsfläche 6 eine Projektionsleinwand verwendet. Der Projektor weist weitere Anschlüsse für andere Datensignalquellen als die Kamera auf.

Die Bedieneinheit 11 weist Bedienelemente für Einstellungen auf, die auch direkt an dem Projektor 4 und der Kamera 7 vorgenommen werden können. Insofern übernimmt die Bedieneinheit 11 die Funktion einer Fernbedienung. Die einzelnen Bedienelemente sind durch Größe und Übersichtlichkeit ihrer Anordnung so optimiert, daß sie eine Bedienung durch Sehbehinderte ermöglichen. Weiter ist die Bedieneinheit 11 so ausgebildet, daß die Bedienelemente für den Sehbehinderten auch über den Tastsinn erfaßbar sind. Die Bedieneinheit 11 besitzt insbesondere einen Ein/Aus-Schalter für Projektor 4 und Kamera 7, Einstellmöglichkeiten für Helligkeit und Kontrast der Abbildung, einen Schalter zum Ein- und Ausschalten von Kontrastumkehr, einen Schalter für wahlweise Schwarz/Weiß- oder Farbdarstellung, und einen Schalter zum Anwählen einer Datensignalquelle einschließlich der Kamera 7.

Um das Videosignal von der Kamera 7 an den Projektor 4 zu übertragen, weist die Kamera 7 einen Sender auf, während der Projektor 4 einen entsprechenden Empfänger aufweist. Für die Übertragung von Kontrollsignalen zwischen dem Bedienelement einerseits und der Kamera 7 und dem Projektor 4 andererseits sind Infrarotschnittstellen vorgesehen.

Der Betrieb der zuvor beschriebenen elektronischen Sehhilfe ist wie folgt:

Vor der ersten Inbetriebnahme werden einmalig der Abstand zwischen Projektor 4 und Projektionsfläche 6 sowie der Abstand zwischen der Projektionsfläche 6 und dem Benutzer 12 eingestellt. Dabei legt der Abstand zwischen Projektor 4 und Projektionsfläche 6 die Größe der Abbildung und der Abstand zwischen Projektionsfläche 6 und Benutzer 12 den Betrachtungswinkel fest. Um Objekte, wie zum Beispiel eine Schrift, Hände oder anderes mit der weiter oben beschriebenen elektronischen Sehhilfe zu betrachten, werden diese zu betrachtenden Objekte 3 von dem Benutzer in den Aufnahmebereich der Kamera 7 gehalten. Der Benutzer 12 nimmt dann über die einzelnen Bedienelemente der Bedieneinheit 11 Einstellungen hinsichtlich der einzelnen Abbildungsparameter wie Kontrast, Helligkeit und Vergrößerung vor, so daß ein von dem Projektor 4 auf die Projektionsfläche 6 abgebildetes Objekt für ihn optimal wahrnehmbar ist. Um verschiedene Abschnitte des Gegenstands 3 abzubilden, kann der Benutzer entweder den Gegenstand 3 durch den Aufnahmebereich der Kamera 7 bewegen oder die Ausrichtung der Kamera 7 verstellen. Der Sehbehinderte 12 blickt auf eine ihm gegenüberliegende Projektionsfläche 6, auf der er ein vergrößertes Abbild des zu betrachtenden Objekts 3 sehen kann.

Die folgende Beispielrechnung für den Betrachtungswinkel eines bekannten elektronischen Bildschirmlesegeräts und der erfindungsgemäßen elektronischen Sehhilfe macht den Vorteil der Erfindung deutlich. Die übliche Höhe eines Monitors (entspricht der maximal darstellbaren Buchstabenhöhe) beträgt in etwa 30 cm. Bei einer üblichen Betrachtungsentfernung von 40 cm ergibt sich damit ein Betrachtungswinkel von

$$2 \arctan (15/40) = 41°$$

für das bekannte elektronische Bildschirmlesegerät. Die Höhe der erfindungsgemäßen Projektionsfläche beträgt üblicherweise 100 cm, während die Betrachtungsentfernung üblicherweise bei 100 cm liegt. Damit ergibt sich ein Betrachtungswinkel von

$$2 \arctan (20/40) = 53°.$$

Diese Rechnung zeigt, daß mit der erfindungsgemäßen elektronischen Sehhilfe größere Betrachtungswinkel erreicht werden können als mit den bekannten elektronischen Bildschirmlesegeräten.

In einer Abwandlung der oben beschriebenen Ausführungsform ist der Bildausschnitt eines dargestellten Gegenstands über die Bedieneinheit 11 anwählbar. Dies kann zum Beispiel mit einem Joystick, einer Maus, Pfeiltasten oder dergleichen realisiert werden. Von der Kamera 7 wird dabei ein bestimmter Ausschnitt dieses von der Kamera 7 erfaßten Bereichs ausgewählt, der dann in vergrößerter Form auf der Projektionsfläche 6 abgebildet wird.

In einer weiteren Abwandlung besitzt die Kamera 7 ein elektrisches oder optisches Zoomsystem, über das die Vergrößerung realisiert werden kann.

In der oben beschriebenen Ausführungsform ist der Projektor 4 an der Decke 10 montiert, jedoch kann der Projektor auch platzsparend an einer Wand 5 montiert oder aber einfach auf eine geeignete Unterlage gestellt werden.

In der oben beschriebenen Ausführungsform ist die Kamera 7 an einem Stativ 8 befestigt, jedoch kann die Kamera in einer weiteren Abwandlung auch als Handkamera ausgebildet sein.

In der oben beschriebenen Ausführungsform wird die Signal- und Datenübertragung schnurlos über Funk- bzw. Infrarotverbindung beschrieben, jedoch kann die Übertragung auch über Kabel erfolgen.

Anstelle einer digitalen Kamera 7 kann auch eine Kamera 7 mit einem analogen Ausgangssignal verwendet werden. Entsprechend wird dann ein Projektor mit einem analogen Videoeingang verwendet werden.

Anstelle des von der Kamera 7 erfaßten Bildes

kann auch ein Fernseh-, Video- oder ein Computerbild an den Projektor übertragen werden. Somit können auch diese von anderen Datensignalquellen als der Kamera 7 stammenden Bilder elektronisch verarbeitet und in vergrößerter, für Sehbehinderte geeigneter Form dargestellt werden.

**Patentansprüche**

1. Elektronische Sehhilfe für Sehbehinderte mit einer von einer Halteeinrichtung (8) gehaltenen Kamera (7) und einem Projektor (4), der das von der Kamera (7) erfaßte Bild in Echtzeit vergrößert auf einer Projektionsfläche (6) abbildet.

2. Elektronische Sehhilfe nach Anspruch 1, weiter mit einer Bedieneinheit (11) zur Fernbedienung von Einstellungen an der Kamera (7) und dem Projektor (4).

3. Elektronische Sehhilfe nach Anspruch 1 oder 2, bei der die Kamera (7) eine digitale Kamera (7) ist und der Projektor (4) einen digitalen Videoeingang aufweist.

4. Elektronische Sehhilfe nach einem der Ansprüche 1 bis 3, bei der die Kamera (7) über einen Autofokus verfügt.

5. Elektronische Sehhilfe nach einem der Ansprüche 1 bis 4, bei der die Halteeinrichtung (8) für die Kamera (7) derart ausgebildet ist, daß die Kamera (7) in alle Richtungen ausgerichtet werden kann.

6. Elektronische Sehhilfe nach einem der Ansprüche 1 bis 5, bei der die Halteeinrichtung (8) für die Kamera (7) eine Tischklemme oder einen Standfuß aufweist.

7. Elektronische Sehhilfe nach einem der Ansprüche 1 bis 6, bei der in der Kamera (7) oder dem Projektor (4) eine Vorrichtung zur elektronischen Bildverarbeitung vorgesehen ist.

8. Elektronische Sehhilfe nach einem der Ansprüche 1 bis 7, bei der der Projektor (4) weitere Datensignaleingänge zum Anschluß von anderen Datensignalquellen als der Kamera (7) aufweist.

9. Elektronische Sehhilfe nach einem der Ansprüche 1 bis 8, **dadurch gekennzeichnet, daß** der Projektor ein CRT- bzw. Kathodenstrahl-Beamer ist.

10. Elektronische Sehhilfe nach einem der Ansprüche 1 bis 8, **dadurch gekennzeichnet, daß** der Projektor ein LCD- bzw. Flüssigkristall-Beamer ist.

11. Elektronische Sehhilfe nach einem der Ansprüche 1 bis 8, **dadurch gekennzeichnet, daß** der Projektor ein DLP- bzw. Spiegel-Beamer ist.

12. Elektronische Sehhilfe nach einem der Ansprüche 1 bis 11, bei der das Bedienteil (11) ein Bedienelement zur Auswahl eines Bildausschnitts aufweist.

13. Elektronische Sehhilfe nach einem der Ansprüche 1 bis 12, bei der das Bedienteil ein Bedienelement zur Einstellung der Bildvergrößerung aufweist.

14. Elektronische Sehhilfe nach einem der Ansprüche 1 bis 13, bei der das Bedienteil ein Bedienelement zur Einstellung der Hintergrundfarbe aufweist.

15. Elektronische Sehhilfe nach einem der Ansprüche 1 bis 14, bei der das Bedienteil (11) ein Bedienelement zum Umschalten zwischen Schwarz/Weiß- und Farbdarstellung aufweist.

16. Elektronische Sehhilfe nach einem der Ansprüche 1 bis 15, bei der das Bedienteil (11) ein Bedienelement zur Einstellung des Kontrasts, insbesondere der Kontrastumkehr und der Kontrasterstärkung aufweist.

17. Elektronische Sehhilfe nach einem der Ansprüche 4 bis 16, **dadurch gekennzeichnet, daß** die Kamera eine analoge Kamera ist.

18. Elektronische Sehhilfe nach einem der Ansprüche 1 bis 17, **dadurch gekennzeichnet, daß** eine Signalübertragungseinrichtung zwischen der Kamera und dem Projektor vorgesehen ist, die als kabelloses Netz ausgebildet ist.

19. Elektronische Sehhilfe nach einem der Ansprüche 1 bis 18, **dadurch gekennzeichnet, daß** der Projektor und die Kamera über ein Kabel verbunden sind.

FIGUR 1

FIGUR 2

**Europäisches Patentamt**

## EUROPÄISCHER RECHERCHENBERICHT

Nummer der Anmeldung

EP 04 00 4910

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 00 64140 A (INNOVENTIONS INC)<br>26. Oktober 2000 (2000-10-26)<br>* das ganze Dokument *<br>--- | 1-19 | H04N7/18 |
| X<br><br>A | US 2002/135674 A1 (ANDO TAKAHISA ET AL)<br>26. September 2002 (2002-09-26)<br>* Absätze<br>[0002]-[0004],[0017],[0018],[0027]-[0030]<br>*<br>--- | 1,3,<br>5-15,19<br>2,4,<br>16-18 | |
| X | US 5 767 905 A (ARCHAMBO MICHAEL)<br>16. Juni 1998 (1998-06-16)<br>* Spalte 4, Zeile 24 - Spalte 5, Zeile 65<br>*<br>--- | 1,2,5,6 | |
| X | DE 199 52 393 A (BAUM RETEC AG)<br>15. März 2001 (2001-03-15)<br>* das ganze Dokument *<br>--- | 1-19 | |
| X | DE 197 34 902 A (ACRITEC GMBH)<br>30. Juli 1998 (1998-07-30)<br>* Spalte 2, Zeile 56 - Spalte 3, Zeile 47<br>*<br>----- | 1,17 | |

RECHERCHIERTE SACHGEBIETE (Int.Cl.7)

H04N
G09B

Der vorliegende Recherchenbericht wurde für alle Patentansprüche erstellt

| Recherchenort | Abschlußdatum der Recherche | Prüfer |
|---|---|---|
| MÜNCHEN | 8. April 2004 | Noll, B |

EPO FORM 1503 03.82 (P04C03)

**EP 1 482 739 A1**

## ANHANG ZUM EUROPÄISCHEN RECHERCHENBERICHT
## ÜBER DIE EUROPÄISCHE PATENTANMELDUNG NR.

EP 04 00 4910

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-04-2004

| Im Recherchenbericht angeführtes Patentdokument | | Datum der Veröffentlichung | Mitglied(er) der Patentfamilie | | Datum der Veröffentlichung |
|---|---|---|---|---|---|
| WO 0064140 | A | 26-10-2000 | AU | 4201800 A | 02-11-2000 |
| | | | CA | 2369982 A1 | 26-10-2000 |
| | | | EP | 1171993 A1 | 16-01-2002 |
| | | | WO | 0064140 A1 | 26-10-2000 |
| US 2002135674 | A1 | 26-09-2002 | JP | 2002287079 A | 03-10-2002 |
| US 5767905 | A | 16-06-1998 | KEINE | | |
| DE 19952393 | A | 15-03-2001 | DE | 19952393 A1 | 15-03-2001 |
| | | | EP | 1096779 A2 | 02-05-2001 |
| DE 19734902 | A | 30-07-1998 | DE | 19734902 A1 | 30-07-1998 |

EPO FORM P0461

Für nähere Einzelheiten zu diesem Anhang : siehe Amtsblatt des Europäischen Patentamts, Nr.12/82

8